# EUROPÄISCHE PATENTSCHRIFT

(11) **EP 1 620 373 B1**
(45) Veröffentlichungstag und Bekanntmachung des Hinweises auf die Patenterteilung: **26.03.2008**
(21) Anmeldenummer: 04731136.0
(22) Anmeldetag: 05.05.2004
(51) Int. Cl.: C07B 59/00

(54) **VORRICHTUNG UND VERFAHREN ZUM NUCLEOPHILEN FLUORIEREN**
DEVICE AND METHOD FOR THE FLUORINATION OF NUCLEOPHILES
DISPOSITIF ET PROCEDE DE FLUORATION NUCLEOPHILE

(30) Priorität: 07.05.2003 DE 10320552; 09.05.2003 US 468963 P
(43) Veröffentlichungstag der Anmeldung: 01.02.2006
(73) Patentinhaber: Bayer Schering Pharma Aktiengesellschaft, 13353 Berlin (DE)
(72) Erfinder: MAEDING, Peter, 01277 Dresden (DE); FUECHTNER, Frank, 01324 Dresden (DE)
(74) Vertreter: Bittner, Thomas L.
(86) Internationale Anmeldenummer: PCT/EP2004/004784
(87) Internationale Veröffentlichungsnummer: WO 2004/100490

(56) Entgegenhaltungen:
- EP-A- 0 949 632
- US-A- 5 932 178
- TOORONGIAN S A ET AL: "ROUTINE PRODUCTION OF 2-DEOXY-2- 18FFLUORO-D-GLUCOSE BY DIRECT NUCLEOPHILIC EXCHANGE ON A QUATERNARY 4-AMINOPYRIDINIUM RESIN" INTERNATIONAL JOURNAL OF RADIATION APPLICATIONS AND INSTRUMENTATION PART B: NUCLEAR MEDICINE AND BIOLOGY, ELSEVIER SCIENCE PUBLISHERS, NEW YORK, NY, US, Bd. 17, Nr. 3, 1990, Seiten 273-279, XP000676335 ISSN: 0883-2897

## Beschreibung

### Bereich der Erfindung

Die Erfindung liegt auf dem Gebiet der nucleophilen Fluorierung einer Substanz, insbesondere zur Synthese einer ¹⁸F-markierten Substanz für eine Untersuchung mit Hilfe eines Positronen-Emissions-Tomographen.

### Hintergrund der Erfindung

Die Positronen-Emissions-Tomographie (PET) ist eine nukleare Medizintechnik, bei der radiopharmazeutische Mittel genutzt werden, die aus biologisch relevanten Molekülen, welche mit Positronen emittierenden Isotopen markiert sind, gebildet werden. PET wird verwendet, um Stoffwechselprozesse und physiologische Vorgänge zu untersuchen. Aufgrund der Nutzung der Analyse der Strahlung von kurzlebigen Radioisotopen von Elementen, die sich im menschlichen Körper befinden, liefert PET zusätzliche Informationen zu anderen diagnostischen Verfahren wie beispielsweise die Computer basierte Tomographie oder Untersuchungen mit Hilfe von Magnetresonanz. PET-Radiopharmaka nehmen an biochemischen Reaktionen des Körpers mit einer für den Menschen nicht kritischen Dosis teil.

Die Nutzbarkeit von PET hängt wesentlich von der Verfügbarkeit nichttoxischer Radiopharmaka ab. Als eines der bevorzugten Radioisotope hat sich Fluor-18 (¹⁸F) erwiesen, weil seine Zerfallsenergie von 0,64 MeV eine hohe inhärente Auflösung während PET-Messungen ermöglicht. ¹⁸F hat darüber hinaus eine vorteilhafte Halbwertszeit von 109,8 min. Erfolgreich angewendet wurde bisher insbesondere 2-[¹⁸F]Fluor-2-desoxy-D-glucose ([¹⁸F]FDG). Diese Markierungssubstanz wird weltweit für verschiedenste Anwendungen genutzt. [¹⁸F]FDG ist eine mit ¹⁸F markierte Zuckerverbindung, die einem Patienten ohne weiteres verabreicht werden kann. [¹⁸F]FDG wird von wachsenden Krebszellen, dem Gehirn oder Herzmuskeln ohne weiteres verarbeitet. Die beschriebenen Eigenschaften von [¹⁸F]FDG haben dazu geführt, daß es erfolgreich in der Nuklearmedizin eingesetzt wird. Die Nutzung von PET in klinischen Anwendungen hat dazu geführt, daß Vorrichtungen zum Synthetisieren von Radiopharmaka wie [¹⁸F]FDG entwickelt wurden.

Die Veröffentlichung von N. Satyamurthy: Electronic Generators for the Production of Positron-Emitter labeled Radiopharmaceuticals: Where would PET be without them?, Clinical Positron Imaging, Vol. 2, Nr. 5, Seiten 233-253, 1999, beschreibt überblicksartig verschiedene Vorrichtungen zur automatisierten FDG-Synthese.

In der Druckschrift US 5,932,178 ist ein FDG-Synthesemodul mit einer Säule offenbart, die mit einem polymergestützten Katalyseharz gefüllt ist. In der Druckschrift US 5,808,020 sind eine optische Reaktionszelle und eine Lichtquelle für Verfahren zur Synthese von ¹⁸F-markierten eines Radiotracern unter Verwendung von [¹⁸F]Fluorid beschrieben.

In der Veröffentlichung S. A. Toorongian et. al: "Routine Production of 2-Deoxy-2-[18F]fluoro-D-glucose by Direct Nucleophilic Exchange on a Quatemary 4-Aminopyridinium Resin", Nucl. Med. Biol., Vol. 17, Nr. 3, ist ein Verfahren zum nucleophilen Fluorieren beschrieben. Der Verlauf der Markierungsreaktion wird mittels Beobachten der Abnahme der Radioaktivität in einer Ionisationskammer beobachtet.

In der Druckschrift WO 02/36581 werden neuartige radiopharmazeutische Mittel beschrieben, die an den CCR1-Rezeptor binden, welcher in Verbindung mit der Alzheimer Krankheit in Gehirnbereichen von Patienten auftritt.

Aufgrund der Suche nach neuartigen geeigneten Radiopharmaka, die auf neuen Syntheseverfahren beruhen, besteht Bedarf für Vorrichtungen, die zur Synthese der Radiopharmaka genutzt werden können.

### Die Erfindung

Aufgabe der Erfindung ist es deshalb, eine verbesserte Vorrichtung und eine verbessertes Verfahren zum nucleophilen Fluorieren anzugeben, die (das) eine anwendungsabhängige Synthese von nucleophil fluorierten Substanzen in einer Art und Weise ermöglicht, die für flexible klinische Anwendungen geeignet ist.

Diese Aufgabe wird erfindungsgemäß durch eine Vorrichtung nach dem unabhängigen Anspruch 1 und ein Verfahren nach dem unabhängigen Anspruch 8 gelöst.

Eine zum nucleophilen Fluorieren einer Substanz genutzte Vorrichtung, insbesondere zur Synthese einer ¹⁸F-markierten Substanz für eine Untersuchung mit Hilfe eines Positronen-Emissions-Tomographen, umfaßt eine Anionen-Austauscheinrichtung zum Extrahieren von [¹⁸F]Fluoridionen mittels Adsorption aus einem Target-Fluid, wobei die Anionen-Austauscheinrichtung über eine Zuführeinrichtung mit dem Target-Fluid beladen werden kann, und einer Meßeinrichtung mit einer Meßkammer zum Messen einer Ausgangsradioaktivität der [¹⁸F]Fluoridionen. Hierbei ist die Anionen-Austauscheinrichtung zumindest teilweise in der Meßkammer der Meßeinrichtung angeordnet. Dieses hat den Vorteil, daß die Ausgangsradioaktivität der [¹⁸F]Fluoridionen gemessen werden kann, während sich diese in der Anionen-Austauscheinrichtung befinden. Es treten keine Verluste durch ein zusätzliches Sammelgefäß für das Target-Fluid auf.

Es ist ein Gefäß zum Aufnehmen eines nucleophil fluorierten Reaktionsproduktes vorgesehen, wobei das Gefäß zumindest teilweise in der Meßkammer der Meßeinrichtung angeordnet ist, um eine Radioaktivität des nucleophil fluorierten Reaktionsproduktes zu messen. Auf diese Weise können die Ausgangsradioaktivität und die Radioaktivität des Reaktionsproduktes mit Hilfe einer einzelnen Meßeinrichtung gemessen werden. Beide Messungen können ausgeführt werden, ohne daß Teile der Vorrichtung zum Messen verlagert oder umgeordnet werden müssen.

Die Genauigkeit der Radioaktivitätsmessungen wird bei einer zweckmäßigen Ausgestaltung der Erfindung dadurch verbessert, daß die Meßeinrichtung eine kalibrierbare Meßeinrichtung ist. Dieses hat darüber hinaus den Vorteil, daß bei einer Folgemessung eine Untergrundradioaktivität aufgrund von Rückständen in der Meßkammer mittels Kompensation der Untergrundradioaktivität als Meßwertverfälschung beseitigt werden kann.

Eine kompakt ausführbare Meßeinrichtung, die mit der notwendigen Genauigkeit ausgestattet ist, kann bei einer vorteilhaften Weiterbildung der Erfindung dadurch gebildet werden, daß die Meßeinrichtung ein Aktivimeter ist. Ein Aktivimeter dient der genauen und schnellen Bestimmung der Radioaktivität von Radionukliden. Wesentliche Vorteile der Radioaktivitätsmessung mit einem Aktivimeter bestehen in der 4-π-Meßgeometrie, dem großen linearen Messbereich und der nuklidspezifischen Kalibrierung.

Die Nutzung der Vorrichtung für eine nucleophile Fluorierung, bei der eine möglichst große Reinheit des Reaktionsproduktes notwendig ist, wird bei einer vorteilhaften Ausführungsform der Erfindung dadurch ermöglicht, daß eine HPLC-Einrichtung (HPLC-"High-Performance Liquid Chromatography) mit einer HPLC-Säule zum Reinigen eines Reaktionsgemisches vorgesehen ist. Eine solche auch als präparative HPLC bezeichnete HPLC-Einrichtung dient zur Isolation und Reinigung von Komponenten. Bei nucleophilen Reaktionen treten häufig Reaktionsgemische auf, welche mit Hilfe der HPLC-Einrichtung aufzutrennen sind.

Eine bevorzugte Weiterbildung der Erfindung kann vorsehen, daß die HPLC-Einrichtung ein mit einer Kopplungsleitung zum Beladen einer Dosiereinrichtung in Verbindung stehendes Probenaufgabeventil umfaßt; das Probenaufgabeventil über eine Abfallleitung an einen Abfallbehälter gekoppelt ist; dem Probenaufgabeventil zum Detektieren des Reaktionsgemischs in der Kopplungsleitung eine Fluidsensoreinrichtung vorgeschaltet ist; und das Probenaufgabeventil steuerbar ausgeführt ist, so daß das Probenaufgabeventil in einem Ausgangszustand eingestellt ist, um über die Dosiereinrichtung eine Fluidverbindung zwischen der Kopplungsleitung und der Abfallleitung zu bilden, wenn mit Hilfe der Fluidsensoreinrichtung das Reaktionsgemisch in der Zuführleitung detektiert wird, und das Probenaufgabeventil in einen Injektionszustand zum Beladen der HPLC-Säule umgeschaltet wird, um in dem Injektionszustand eine Fluidverbindung zwischen der Dosiereinrichtung und der HPLC-Säule zu bilden, wenn mit Hilfe der Fluidsensoreinrichtung das Reaktionsgemisch in der Zuführleitung nicht mehr detektiert wird. Mit Hilfe dieser Ausgestaltung wird vermieden, daß beim Beladen der HPLC-Einrichtung mit dem Reaktionsgemisch Luft, welche sich in den der HPLC-Einrichtung vorgeschalteten Leitungen befindet, bevor das Reaktionsgemisch in diese Leitungen gelangt, in die Dosiereinrichtung der HPLC-Einrichtung überführt wird, was die Ausbeute der Isolation und die Effektivität der Trennung in der HPLC-Einrichtung nachteilig beeinflussen könnte.

Eine zweckmäßige Ausgestaltung der Erfindung kann vorsehen, daß mittels der Kopplungsleitung eine direkte Kopplung zwischen der Fluidsensoreinrichtung und einem Reaktionsgefäß gebildet ist. Die direkte Kopplung vermindert die Wahrscheinlichkeit für Verluste beim Übertragen des Reaktionsgemisches.

Eine zweckmäßige Ausführungsform der Erfindung kann vorsehen, daß die HPLC-Einrichtung eine Reinigungseinrichtung mit einer UV-Detektoreinrichtung und einer auf die UV-Detektoreinrichtung folgenden Gamma-Detektoreinrichtung zum Reinigen des Reaktionsgemischs mit Hilfe der UV-Detektoreinrichtung und anschließend mit Hilfe der Gamma-Detektoreinrichtung umfaßt. Durch diese Anordnung lässt sich ein radioaktiver Peak so isolieren, daß möglichst geringe chemische Verunreinigungen mit entsprechender UV-Absorption enthalten sind und die Verluste des radioaktiven Endprodukts minimiert werden können.

Die Merkmale aus den abhängigen Ansprüchen des Verfahrens zum nucleophilen Fluorieren einer Substanz weisen die in Verbindung mit den zugehörigen Merkmalen in den abhängigen Vorrichtungsansprüchen genannten Vorteile entsprechend auf.

### Kurzbeschreibung der Figuren

Die Erfindung wird im folgenden anhand von Ausführungsbeispielen unter Bezugnahme auf eine Zeichnung näher erläutert. Hierbei zeigen:
- Figur 1: eine schematische Darstellung einer Vorrichtung zum nucleophilen Fluorieren einer Substanz;
- Figur 2: ein Syntheseschema für 4-[¹⁸F]FBA (**2**) aus TMABATf (**1**) und [¹⁸F]Fluorid;
- Figur 3: ein Syntheseschema für [18F]ZK811460 (**4**) aus einem Piperazin-Derivat (**3**) und [18F]FBA (**2**); und
- Figur 4: eine schematische Darstellung eines Probenaufgabeventils.

### Detaillierte Beschreibung bevorzugter Ausführungsformen

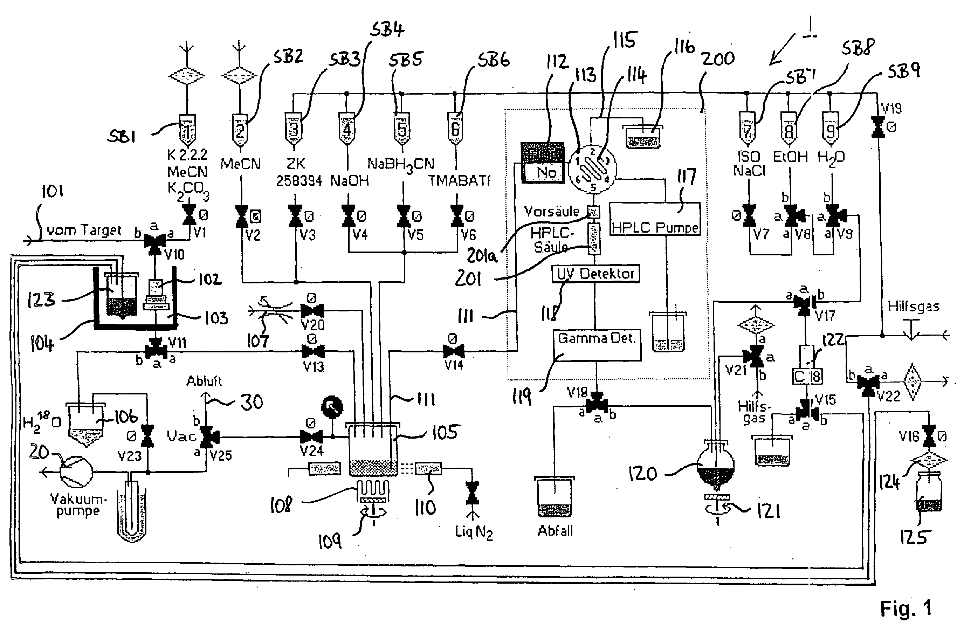

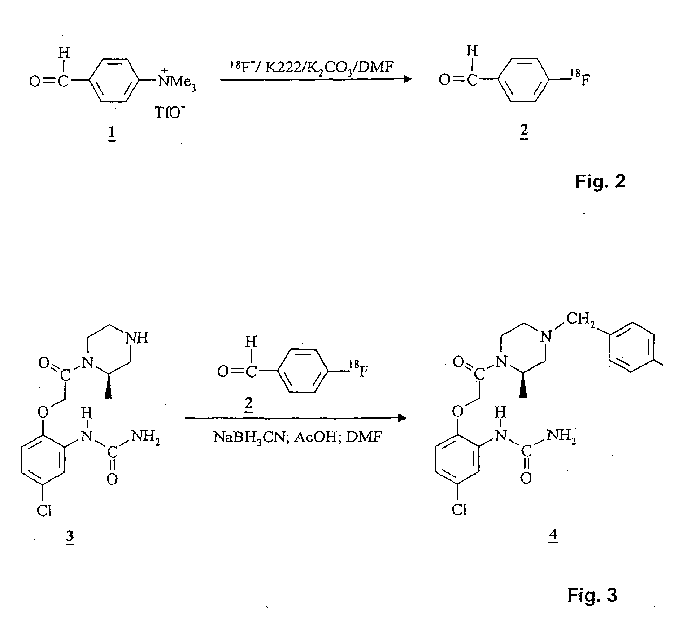

Figur 1 zeigt eine schematische Darstellung einer Vorrichtung 1 zum nucleophilen Fluorieren einer Substanz. Die Nutzung der Vorrichtung 1 zur Synthese wird anhand des Beispiels der Herstellung der markierten Verbindung 1-(5-Chlor-2-{2-[(2R)-4-(4-[¹⁸F]fluorobenzyl)-2-methylpiperazin-1-yl]-2-oxo-ethoxy}phenyl)harnstoff erläutert, was ausgehend von TMABATf (**1**) mittels Radiosynthese von 4-[¹⁸F]FBA (**2**) und dessen reduktiver Aminierung mit einem Piperazin-Derivat (**3**) gebildet wird (vgl. Figuren 2 und 3). Die auf diesem Wege erhaltene markierte Verbindung wird im folgenden kurz als [¹⁸F]ZK811460 (**4**) bezeichnet. Weitere spezifische Einzelheiten, insbesondere hinsichtlich der verwendeten chemischen Substanzen und von Reaktionsparametern, sofern sie sich nicht aus der folgenden Beschreibung ergeben, können der Veröffentlichung Mäding, et al., Annual Report 2002, Institute of Bioinorganic and Radiopharmaceutical Chemistry, FZR-363, 40 entnommen werden und sind für die Verwirklichung der Erfindung in ihren verschiedenen Ausführungsformen unkritisch.

Die in einem Target-Fluid enthaltenen [¹⁸F]Fluoridionen werden über eine Zuführung 101 und ein Ventil V 10 einer Anionen-Austauscheinrichtung 102 zugeführt. Die Anionen-Austauscheinrichtung 102 dient zum Extrahieren von [¹⁸F]Fluoridionen aus dem Target-Fluid. Die Extraktion wird mit Hilfe einer Adsorption ausgeführt. Gemäß Figur 1 ist die Anionen-Austauscheinrichtung 102 in einer Meßkammer 103 einer Meßeinrichtung 104 angeordnet, welche zum Messen der Radioaktivität dient. Die Meßeinrichtung 104 ist hierbei vorzugsweise als ein Aktivimeter ausgebildet. Mit Hilfe der Meßeinrichtung 104 kann die Ausgangsradioaktivität von [¹⁸F]Fluoridionen während und nach deren Extraktion aus dem Target-Fluid mit Hilfe der Anionen-Austauscheinrichtung 102 gemessen werden.

Die Anionen-Austauscheinrichtung 102 ist über ein Ventil V 10 mit der Zuführung 101 sowie einem Ventil V1 verbunden. Über das Ventil V1 können Substanzen zum Ventil V10 abgegeben werden, die in einem Speicherbehälter SB1 vorrätig sind. Die Substanzen werden hierbei mit Hilfe von Vakuum oder hilfsweise mittels eines Gases, beispielsweise Stickstoff, durch Leitungen und Ventile übertragen. Die Anionen-Austauscheinrichtung 102 ist weiterhin an ein Ventil V11 gekoppelt, über welches die extrahierten [¹⁸F]Fluoridionen nach deren Desorption nach dem Passieren eines Ventils V13 in ein Reaktionsgefäß 105 gelangen. Über das Ventil V11 gelangt separiertes [¹⁸O]H₂O durch Öffnen des Ventils V23 bei eingeschalteter Vakuumpumpe 20 aus der Anionen-Austauscheinrichtung 102 in einen Behälter 106. Die Ventile V24 und V25 dienen zum Anlegen von Vakuum an das Reaktionsgefäß 105 bzw. zu dessen Belüftung.

Gemäß Figur 1 ist das Reaktionsgefäß 105 mit weiteren Ventilen V2, V3, V4, V5, und V6 verbunden, die an jeweilige Speicherbehälter SB2-SB6 gekoppelt sind. Über die Ventile V2-V6 können die in den jeweiligen Speicherbehältern SB2-SB6 vorrätigen chemischen Substanzen in vorgegebenem Umfang in das Reaktionsgefäß 105 eingebracht werden, um die gewünschte chemische Reaktion zum Synthetisieren von [¹⁸F]ZK811460 (**4**) auszuführen. Die Substanzen werden hierbei mit Hilfe von Vakuum oder hilfsweise mittels eines Gases, beispielsweise Stickstoff, durch Leitungen und Ventile übertragen. Zum Steuern einer Beaufschlagung des Reaktionsgefäßes 105 mit Schutzgas aus einer Leitung 107 dient ein Ventil V20. Zum Abführen der bei der chemischen Reaktion entstehenden Abgase ist das Reaktionsgefäß 105 weiterhin über ein Ventil V24 und V25 mit einer Abgasöffnung 30 verbunden.

Zur Synthese von [¹⁸F]ZK811460 im Reaktionsgefäß 105 wird zunächst mit einer Lösung von Kryptofix 2.2.2 und Kaliumkarbonat in wäßrigem Acetonitril (aus SB1) von der Anionen-Austauschvorrichtung 102 das [¹⁸F]Fluorid eluiert und mittels Vakuum und Stickstoff-Strom bei 95 °C getrocknet. Eine zusätzliche Trocknung erfolgt mittels Zugabe von wasserfreiem Acetonitril (aus SB2) und dessen Verdampfen. Nach Zugabe einer Lösung von TMABATf (1) in DMF (aus SB6) wird das Reaktionsgemisch 10 min bei 120 °C erhitzt. Dann werden nacheinander eine essigsaure Lösung des Piperazin-Präkursors (**3**) (ZK258394 aus SB3) und eine Lösung von NaBH₃CN in DMF (aus SB5) zugegeben. Nach 10-minütigem Erhitzen bei 120 °C wird die Reaktionsmischung mit wäßriger NaOH (aus SB4) neutralisiert.

Zum Einstellen der gewünschten Reaktionsparameter sind im Bereich des Reaktionsgefäßes 105 eine Heizeinrichtung 108, eine Rühreinrichtung 109 sowie eine Kühleinrichtung 110 vorgesehen.

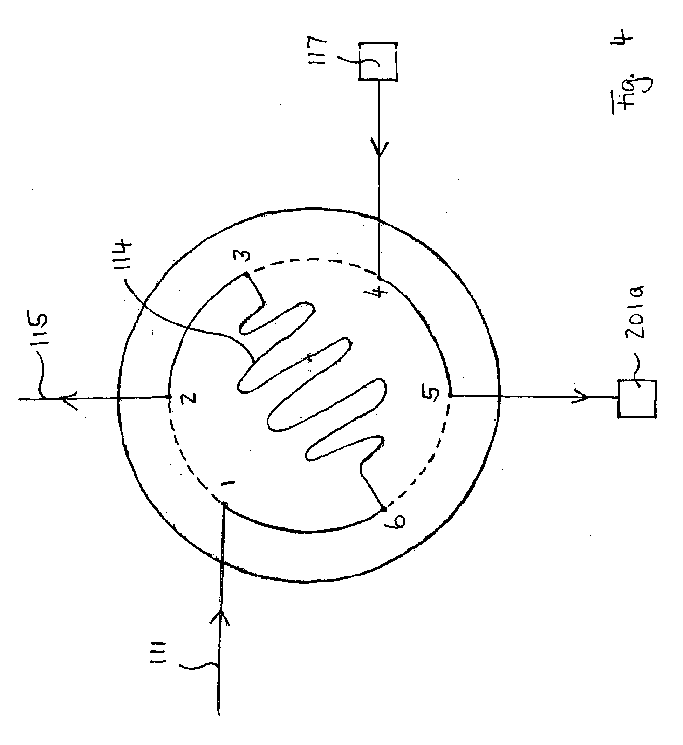

Über eine direkte Kopplungsleitung 111, in welcher ein Ventil V14 angeordnet ist, gelangt das Reaktionsgemisch [¹⁸F]ZK811460 (4) zu einem Fluidsensor 112, welcher ein Fluid in der direkten Kopplungsleitung 111 detektiert. Der Fluidsensor 112 ist einem Injektions- bzw. Probenaufgabeventil 113 unmittelbar vorgeschaltet, dessen Funktionsweise im folgenden unter Bezugnahme auf die Figuren 1 und 4 beschrieben ist, wobei letztere eine schematische Darstellung eines 6-Wege-Probenaufgabeventils zeigt. Mit Hilfe des Probenaufgabeventils 113 wird eine Dosierschleife 114 über die direkte Kopplungsleitung 111 mit dem Reaktiongemisch beladen. Hierbei wird das Probenaufgabeventil 113 so gesteuert, daß zunächst über die Punkte 1 und 2 (vgl. Figur 4) eine Verbindung zwischen der direkten Kopplungsleitung 111 bzw. dem Fluidsensor 112 und einer Abfallleitung 115 gebildet ist, die von dem Probenaufgabeventil 113 zu einem Abfallbehälter 116 führt. Auf diese Weise wird Luft, die sich in der direkten Kopplungsleitung 111 vor dem Reaktionsgemisch befindet, in die Abfallleitung 115 gedrückt. In dieser Stellung des Probenaufgabeventils 113 (Injektionszustand) wird die Dosierschleife 114 von einem HPLC-Eluenten durchspült, wobei der HPLC-Eluent in dem Probenaufgabeventil 113 einen Weg entlang der Punkte 4, 3, 6 und 5 zurücklegt.

Wenn der Fluidsensor 112 die Ankunft des Reaktionsgemisches aus dem Reaktionsgefäß 105 detektiert, wird das Probenaufgabeventil 113 zum Beladen der Dosierschleife 114 in einen Ladezustand umgeschaltet, so daß über die Dosierschleife 114 eine Verbindung zwischen der direkten Kopplungsleitung 111 und der Abfallleitung 115 entlang der Punkte 1, 6, 3 und 2 ausgebildet wird, was in Figur 4 mittels durchgehender Linien dargestellt ist und dazu führt, daß die Dosierschleife 114 mit Reaktionsgemisch beladen wird. Das Volumen der Dosierschleife 114 ist in der Regel größer als das Volumen des Reaktionsgesmisches. Der HPLC-Eluent passiert das Probenaufgabeventil 113 über einen Kurzschluß entlang eines Weges mit den Punkten 4 und 5 in Figur 4. Wird das Reaktionsgemisch nicht mehr von dem Fluidsensor 112 detektiert, schaltet das Probenaufgabeventil 113 erneut in den Injektionszustand um, so daß eine Verbindung entlang des Weges mit den Punkten 4, 3, 6 und 5 (gestrichelte Linie in Figur 4) gebildet ist. Der HPLC-Eluent kann dann das Reaktionsgemisch aus der Dosierschleife 114 auf eine HPLC-Säule 201 mit Vorsäule 201a drücken.

Mit Hilfe der HPLC-Einrichtung 200 wird die Reaktionsmischung gereinigt. Je nach der konkreten Verwendung der Vorrichtung 1 zu verschiedenen Synthesezwecken können die Parameter an der HPLC-Einrichtung 200 dem gewünschten Zweck entsprechend eingestellt und optimiert werden.

Die HPLC-Einrichtung 200 umfaßt in der dargestellten Ausführungsform eine HPLC-Pumpe 117, das Probenaufgabeventil 113 mit der Dosierschleife 114, die HPLC-Säule 201 mit Vorsäule 201a sowie eine UV-Detektoreinrichtung 118 und eine Gamma-Detektoreinrichtung 119, die in Reihe angeordnet sind.

Über ein Ventil V18, einen Mischbehälter 120 mit einer Wasservorlage und einer Rühreinrichtung 121 und ein Ventil V17 gelangt die isolierte Produktfraktion zu einer RP18-Kartusche 122 zum Sammeln des Reaktionsprodukts mittels Festphasen-Extraktion. Nach dem Waschen der Kartusche 122 mit Wasser (aus SB9) wird [¹⁸F]ZK811460 (**4**) mittels Ethanol (aus SB8) eluiert. Die ethanolische Lösung von [¹⁸F]ZK811460 (**4**) wird dann zum Filtern aus einem Elutionsgefäß 123 durch einen sterilisierenden Filter 124 geführt, welcher danach mit einer Injektionslösung auf Salzbasis (aus SB7) gespült wird. Auf diese Weise wird eine klare, sterile, isotonische NaCl-Lösung von [¹⁸F]ZK811460 (**4**) erhalten, die Ethanol enthält und in einem Produktgefäß 125 gesammelt wird. Das Elutionsgefäß 123 ist in der Meßkammer 103 angeordnet, was eine direkte Messung der Radioaktivität des Reaktionsprodukts [¹⁸F]ZK811460 (**4**) ermöglicht. Bei einer bevorzugten kalibrierfähigen Ausführungsform der Meßeinrichtung 104 kann beim Messen der Radioaktivität des Reaktionsproduktes der Radioaktivitätsuntergrund so kompensiert werden, daß eine Restradioaktivität, die nach dem Herauslösen der [¹⁸F]Fluoridionen in der Anionen-Austausch-einrichtung 102 noch vorhanden ist, als Untergrundradioaktivität der auszuführenden Radioaktivitätsmessung des Reaktionsprodukts kompensiert wird. Die Messung der Anfangsradioaktivität in der Anionen-Austauscheinrichtung 102 und der Radioaktivität des Reaktionsproduktes in dem Elutionsgefäß 123 können auf diese Weise mit hoher Genauigkeit mit Hilfe der Meßeinrichtung 104 ausgeführt werden.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Vorrichtung zum nucleophilen Fluorieren einer Substanz, insbesondere zur Synthese einer ¹⁸F-markierten Substanz für eine Untersuchung mit Hilfe eines Positronen-Emissions-Tomographen, mit:
- einer Anionen-Austauscheinrichtung (102) zum Extrahieren von [¹⁸F]Fluoridionen mittels Adsorption aus einem Target-Fluid, wobei die Anionen-Austauscheinrichtung (102) über eine Zuführeinrichtung (101) mit dem Target-Fluid beladen werden kann;
- einem Gefäß (123) zum Aufnehmen eines nucleophil fluorierten Reaktionsproduktes und
- einer Meßeinrichtung (104) mit einer Meßkammer (103) zum Messen einer Radioaktivität;
wobei die Anionen-Austauscheinrichtung (102) zumindest teilweise in der Meßkammer (103) der Meßeinrichtung (104) angeordnet ist, eine Ausgangsradioaktivität der [¹⁸F]Fluoridionen zu messen, und wobei das Gefäß (123) zumindest teilweise in der Meßkammer (103) der Meßeinrichtung (104) angeordnet ist, um eine Radioaktivität des nucleophil fluorierten Reaktionsproduktes zu messen.

2. Vorrichtung nach Anspruch 1, **dadurch gekennzeichnet, daß** die Meßeinrichtung (104) eine kalibrierbare Meßeinrichtung ist.

3. Vorrichtung nach Anspruch 1 oder 2, **dadurch gekennzeichnet, daß** die Meßeinrichtung (104) ein Aktivimeter ist.

4. Vorrichtung nach einem der vorangehenden Ansprüche, **gekennzeichnet durch** eine HPLC-Einrichtung (200) mit einer HPLC-Säule (201) zum Reinigen eines Reaktionsgemischs.

5. Vorrichtung nach Anspruch 4, **dadurch gekennzeichnet, daß**
- die HPLC-Einrichtung (200) ein mit einer Kopplungsleitung (111) zum Beladen einer Dosiereinrichtung (114) in Verbindung stehendes Probenaufgabeventil (113) umfaßt;
- das Probenaufgabeventil (113) über eine Abfallleitung (115) an einen Abfallbehälter (116) gekoppelt ist;
- dem Probenaufgabeventil (113) zum Detektieren des Reaktionsgemischs in der Kopplungsleitung (111) eine Fluidsensoreinrichtung (112) vorgeschaltet ist; und
- das Probenaufgabeventil (113) steuerbar ausgeführt ist, so daß das Probenaufgabeventil (113) in einem Ausgangszustand eingestellt ist, um über die Dosiereinrichtung (114) eine Fluidverbindung zwischen der Kopplungsleitung (111) und der Abfallleitung (115) zu bilden, wenn mit Hilfe der Fluidsensoreinrichtung (112) das Reaktionsgemisch in der Zuführleitung (111) detektiert wird, und das Probenaufgabeventil (113) in einen Injektionszustand zum Beladen der HPLC-Säule (201) umgeschaltet wird, um in dem Injektionszustand eine Fluidverbindung zwischen der Dosiereinrichtung (114) und der HPLC-Säule (201) zu bilden, wenn mit Hilfe der Fluidsensoreinrichtung (112) das Reaktionsgemisch in der Zuführleitung (111) nicht mehr detektiert wird.

6. Vorrichtung nach Anspruch 4 oder 5, **dadurch gekennzeichnet, daß** mittels der Kopplungsleitung (111) eine direkte Kopplung zwischen der Fluidsensoreinrichtung (112) und einem Reaktionsgefäß (105) gebildet ist.

7. Vorrichtung nach einem der Ansprüche 4 bis 6, **dadurch gekennzeichnet, daß** die HPLC-Einrichtung (200) eine Reinigungseinrichtung mit einer UV-Detektoreinrichtung (118) und einer auf die UV-Detektoreinrichtung (118) folgenden Gamma-Detektoreinrichtung (119) zum Reinigen des Reaktionsgemischs mit Hilfe der UV-Detektoreinrichtung (118) und anschließend mit Hilfe der Gamma-Detektoreinrichtung (119) umfaßt.

8. Verfahren zum nucleophilen Fluorieren einer Substanz, insbesondere zur Synthese einer ¹⁸F-markierten Substanz für eine Untersuchung mit Hilfe eines Positronen-Emissions-Tomographen, bei dem:
- mittels Adsorption aus einem Target-Fluid [¹⁸F]Fluoridionen in einer Anionen-Austauscheinrichtung (102) extrahiert werden, wobei die Anionen-Austauscheinrichtung (102) über eine Zuführeinrichtung (101) mit dem Target-Fluid beladen wird; und
- mit Hilfe einer Meßeinrichtung (104) mit einer Meßkammer (103) eine Ausgangsradioaktivität der [¹⁸F]Fluoridionen gemessen wird, während die Anionen-Austauscheinrichtung (102) zumindest teilweise in der Meßkammer (103) der Meßeinrichtung (104) angeordnet ist; und
- eine Radioaktivität eines nucleophil fluorierten Reaktionsproduktes in einem Gefäß (123) zum Aufnehmen des nucleophil fluorierten Reaktionsproduktes gemessen wird, während das Gefäß (123) zumindest teilweise in der Meßkammer (103) der Meßeinrichtung (104) angeordnet ist.

9. Verfahren nach Anspruch 8, **dadurch gekennzeichnet, daß** die Meßeinrichtung (104) zum Messen der Radioaktivität des nucleophil fluorierten Reaktionsproduktes kalibriert wird.

10. Verfahren nach Anspruch 8 oder 9, **dadurch gekennzeichnet, daß** als Meßeinrichtung (104) ein Aktivimeter verwendet wird.

11. Verfahren nach einem der Ansprüche 8 bis 10, **dadurch gekennzeichnet, daß** ein Reaktionsgemisch mit Hilfe einer HPLC-Einrichtung (200), die eine HPLC-Säule (201) umfaßt, gereinigt wird.

12. Verfahren nach Anspruch 11, bei dem:
- die HPLC-Säule (201) über eine Kopplungsleitung (111) und eine Dosiereinrichtung (114) beladen wird;
- ein Probenaufgabeventil (113) der HPLC-Einrichtung (200) über eine Abfallleitung (115) mit einem Abfallbehälter (116) in Verbindung gebracht wird;
- dem Probenaufgabeventil (113) zum Detektieren des Reaktionsgemisches in der Kopplungsleitung (111) eine Fluidsensoreinrichtung (112) vorgeschaltet ist; und
- das Probenaufgabeventil (113) gesteuert wird, so daß das Probenaufgabeventil (113) in einem Ausgangszustand eingestellt wird, um in dem Ausgangszustand über eine Dosiereinrichtung (114) eine Fluidverbindung zwischen der Kopplungsleitung (111) und der Abfallleitung (115) zu bilden, wenn mit Hilfe der Fluidsensoreinrichtung (112) das Reaktionsgemisch in der Zuführleitung (111) detektiert wird, und das Probenaufgabeventil (113) in einen Injektionszustand zum Beladen der HPLC-Säule (201) umgeschaltet wird, um in dem Injektionszustand eine Fluidverbindung zwischen der Dosiereinrichtung (114) und der HPLC-Säule (201) zu bilden, wenn mit Hilfe der Fluidsensoreinrichtung (112) das Reaktionsgemisch in der Zuführleitung (111) nicht mehr detektiert wird.

13. Verfahren nach einem der Ansprüche 8 bis 12, **dadurch gekennzeichnet, daß** ein vorgereinigtes Reaktionsprodukt zum Filtern als ethanolische Lösung durch eine Filtereinrichtung (124) geführt wird und daß die Filtereinrichtung (124) nachfolgend mit einer Injektionslösung gespült wird, um eine injektionsfähige, sterile Reaktionsproduktlösung zu bilden.

## Claims

1. Device for nucleophilic fluorination of a substance, especially for synthesis of an ¹⁸F-labeled substance for examination using a positron emission tomograph, with:
- an anion exchange device (102) for extraction of [¹⁸F]fluoride ions by means of adsorption from a target fluid, whereby the anion exchange device (102) can be charged via a supply device (101) with the target fluid,
- a vessel (123) for holding a nucleophilically fluorinated reaction product, and
- a measurement device (104) with a measurement chamber (103) for measuring radioactivity;
the anion exchange device (102) being located at least partially in the measurement chamber (103) of the measurement device (104) for measuring initial radioactivity of the [¹⁸F]fluoride ions and the vessel (123) being arranged at least partially in the measurement chamber (103) of the measurement device (104) in order to measure the radioactivity of the nucleophilically fluorinated reaction product.

2. Device according to claim 1, **characterized in that** the measurement device (104) is a measurement device that can be calibrated.

3. Device according to one of the preceding claims, wherein the measurement device (104) is an activity meter.

4. Device according to one of the preceding claims, **characterized by** an HPLC device (200) with an HPLC column (201) for purifying a reaction mixture.

5. Device according to claim 4, wherein
- the HPLC device (200) comprises a sample feed valve (113) that is connected to a coupling line (111) for charging a metering device (114);
- the sample feed valve (113) is coupled via a waste line (115) to a waste tank (116);
- a fluid sensor device (112) is connected upstream from the sample feed valve (113) for detecting the reaction mixture in the coupling line (111); and
- the sample feed valve (113) is made to be controllable such that the sample feed valve (113) is set into an initial state in order to form, via the metering device (114), a fluid connection between the coupling line (111) and the waste line (115), when using the fluid sensor device (112), the reaction mixture in the supply line (111) is detected, and the sample feed valve (113) is switched into an injection state for charging the HPLC column (201) in order to form a fluid connection between the metering device (114) and the HPLC column (201) in the injection state, when the reaction mixture is no longer being detected in the supply line (111) using the fluid sensor device (112).

6. Device according to claim 4 or 5, wherein a direct coupling is formed between the fluid sensor device (112) and a reaction vessel (105) by means of the coupling line (111).

7. Device according to one of claims 4 to 6, wherein the HPLC device (200) comprises a purification device with a UV detector device (118) and a gamma detector device (119) that follows the UV detector device (118) for purifying the reaction mixture using the UV detector device (118) and then using the gamma detector device (119).

8. Process for nucleophilic fluorination of a substance, especially for synthesis of an ¹⁸F-labeled substance for examination using a positron emission tomograph, in which:
- in an anion exchange device (102), [¹⁸F]fluoride ions are extracted by means of adsorption from a target fluid, whereby the anion exchange device (102) is charged via a supply device (101) with the target fluid; and
- using a measurement device (104) with a measurement chamber (103), an initial radioactivity of the [¹⁸F]fluoride ions is measured, while the anion exchange device (102) is located at least partially in the measurement chamber (103) of the measurement device (104),
- and radioactivity of a nucleophilically fluorinated reaction product is measured in a vessel (123) for holding the nucleophilically fluorinated reaction product, while the vessel (123) is located at least partially in the measurement chamber (103) of the measurement device (104).

9. Process according to claim 8, wherein the measurement device (104) for measuring the radioactivity of the nucleophilically fluorinated reaction product is calibrated.

10. Process according to one of claims 8 or 9, wherein an activity meter is used as the measurement device (104).

11. Process according to one of claims 8 to 10, wherein a reaction mixture is purified using an HPLC device (200) that comprises an HPLC column (201).

12. Process according to claim 11, in which:
- the HPLC column (201) is charged via a coupling line (111) and a metering device (114);
- a sample feed valve (113) of the HPLC device (200) is connected via a waste line (115) to a waste tank (116);
- a fluid sensor device (112) is connected upstream from the sample feed valve (113) for detecting the reaction mixture in the coupling line (111); and
- the sample feed valve (113) is controlled such that the sample feed valve (113) is set in an initial state in order to form, via a metering device (114), a fluid connection between the coupling line (111) and the waste line (115) in the initial state, when using the fluid sensor device (112), the reaction mixture in the supply line (111) is detected, and the sample feed valve (113) is switched into an injection state for charging the HPLC column (201) in order to form a fluid connection between the metering device (114) and the HPLC column (201) in the injection state, when using the fluid sensor device (112), the reaction vessel is no longer being detected in the supply line (111).

13. Process according to one of claims 8 to 12, wherein a prepurified reaction product for filtering is routed as an ethanolic solution through a filter device (124) and wherein the filter device (124) is flushed afterwards with an injection solution in order to form an injectable, sterile reaction product solution.

## Revendications

1. Dispositif pour la fluoration nucléophile d'une substance, en particulier pour la synthèse d'une substance marquée ¹⁸F destinée à réaliser une analyse à l'aide d'un tomographe par émission de positrons, avec :
- un équipement échangeur d'anions (102) pour extraire des ions fluorures [¹⁸F] par adsorption d'un fluide cible, étant donné que l'équipement échangeur d'anions (102) peut être alimenté avec le fluide cible par l'intermédiaire d'un système d'alimentation (101) ;
- un récipient (123) pour recueillir un produit de réaction fluoré nucléophile et
- un système de mesure (104) avec une caméra photogrammétrique (103) pour mesurer une radioactivité ;
étant donné que l'équipement échangeur d'anions (102) est disposé au moins en partie dans la caméra photogrammétrique (103) du système de mesure (104) pour mesurer une radioactivité initiale des ions fluorures [¹⁸F], et étant donné que le récipient (123) est disposé au moins en partie dans la caméra photogrammétrique (103) du système de mesure (104) pour mesurer une radioactivité du produit de réaction fluoré nucléophile.

2. Dispositif selon la revendication 1, **caractérisé en ce que** le système de mesure (104) est un système de mesure calibrable.

3. Dispositif selon la revendication 1 ou 2, **caractérisé en ce que** le système de mesure (104) est un activimètre.

4. Dispositif selon l'une quelconque des revendications précédentes, **caractérisé par** un système HPLC (200) avec une colonne HPLC (201) pour purifier un mélange réactionnel.

5. Dispositif selon la revendication 4, **caractérisé en ce que**
- le système HPLC (200) comprend une vanne de distribution d'échantillons (113) reliée à une conduite de connexion (111) pour alimenter un dispositif de dosage (114) ;
- la vanne de distribution d'échantillons (113) est raccordée à un réservoir de déchets (116) par l'intermédiaire d'une conduite de déchets (115) ;
- un équipement détecteur de fluide (112) est placé en amont de la vanne de distribution d'échantillons (113) pour détecter le mélange réactionnel dans la conduite de connexion (111) ; et
- la vanne de distribution d'échantillons (113) est conçue de façon à pouvoir être commandée de telle sorte que la vanne de distribution d'échantillons (113) est réglée à un état initial pour établir, par l'intermédiaire du dispositif de dosage (114), une liaison hydraulique entre la conduite de connexion (111) et la conduite de déchets (115) lorsque, à l'aide de l'équipement détecteur de fluide (112), le mélange réactionnel est détecté dans la conduite de connexion (111) et la vanne de distribution d'échantillons (113) est commutée à un état d'injection pour, étant à cet état d'injection, alimenter la colonne HPLC (201) afin d'établir une liaison hydraulique entre le dispositif de dosage (114) et la colonne HPLC (201) lorsque, à l'aide de l'équipement détecteur de fluide (112), le mélange réactionnel n'est plus détecté dans la conduite d'amenée (111).

6. Dispositif selon la revendication 4 ou 5, **caractérisé en ce qu'une** liaison directe entre l'équipement détecteur de fluide (112) et un récipient de réaction (105) est établie au moyen de la conduite de connexion (111).

7. Dispositif selon l'une quelconque des revendications 4 à 6, **caractérisé en ce que** le système HPLC (200) comprend un système de purification avec un équipement détecteur de rayons UV (118) et un équipement détecteur de rayons gamma (119) placé après l'équipement détecteur de rayons UV (118) pour purifier le mélange réactionnel à l'aide de l'équipement détecteur de rayons UV (118), et ensuite à l'aide du détecteur gamma (119).

8. Procédé pour la fluoration nucléophile d'une substance, en particulier pour la synthèse d'une substance marquée ¹⁸F destinée à réaliser une analyse à l'aide d'un tomographe par émission de positrons, pour lequel :
- des ions fluorures [¹⁸F] sont extraits par adsorption d'un fluide cible dans un équipement échangeur d'anions (102), étant donné que l'équipement échangeur d'anions (102) est alimenté avec le fluide cible par l'intermédiaire d'un système d'alimentation (101) ; et
- à l'aide d'un système de mesure (104), une radioactivité initiale des ions fluorures [¹⁸F] est mesurée avec une caméra photogrammétrique (103), l'équipement échangeur d'anions (102) étant disposé au moins en partie dans la caméra photogrammétrique (103) du système de mesure (104) ; et
- une radioactivité d'un produit de réaction fluoré nucléophile est mesurée dans un récipient (123) destiné à recueillir le produit de réaction fluoré nucléophile, le récipient (123) étant disposé au moins en partie dans la caméra photogrammétrique (103) du système de mesure (104).

9. Procédé selon la revendication 8, **caractérisé en ce que** le système de mesure (104) est calibré pour mesurer la radioactivité du produit de réaction fluoré nucléophile.

10. Procédé selon la revendication 8 ou 9, **caractérisé en ce qu'un** activimètre est utilisé comme système de mesure (104).

11. Procédé selon l'une quelconque des revendications 8 à 10, **caractérisé en ce qu'un** mélange réactionnel est purifié à l'aide d'un système HPLC (200) qui comprend une colonne HPLC (201).

12. Procédé selon la revendication 11, pour lequel :
- la colonne HPLC (201) est alimentée par une conduite de connexion (111) et un dispositif de dosage (114) ;
- une vanne de distribution d'échantillons (113) du système HPLC (200) est raccordée à un réservoir de déchets (116) par une conduite de déchets (115) ;
- un équipement détecteur de fluide (112) est placé en amont de la vanne de distribution d'échantillons (113) pour détecter le mélange réactionnel dans la conduite de connexion (111) ; et
- la vanne de distribution d'échantillons (113) est commandée de telle sorte que la vanne de distribution d'échantillons (113) est réglée à un état initial pour, étant à cet état initial, établir par l'intermédiaire du dispositif de dosage (114) une liaison hydraulique entre la conduite de connexion (111) et la conduite de déchets (115) lorsque, à l'aide de l'équipement détecteur de fluide (112), le mélange réactionnel est détecté dans la conduite de connexion (111) et la vanne de distribution d'échantillons (113) est commutée à un état d'injection pour, étant à cet état d'injection, alimenter la colonne HPLC (201) afin d'établir une liaison hydraulique entre le dispositif de dosage (114) et la colonne HPLC (201) lorsque, à l'aide de l'équipement détecteur de fluide (112), le mélange réactionnel n'est plus détecté dans la conduite d'amenée (111).

13. Procédé selon l'une quelconque des revendications 8 à 12, **caractérisé en ce qu'un** produit de réaction préalablement purifié est conduit à travers un équipement de filtration (124) pour être filtré comme solution éthanolique, et **en ce que** l'équipement de filtration (124) est rincé par la suite avec une solution d'injection pour former une solution de produit de réaction stérile injectable.
